# EUROPEAN PATENT APPLICATION

(11) **EP 2 745 708 A1**
(43) Date of publication of application: **25.06.2014**
(21) Application number: 12382531.7
(22) Date of filing: 24.12.2012
(51) Int. Cl.: A23L 1/305, A23L 1/29, A61K 31/19, A61P 25/24

(54) **Antidepressant effect of ß-hydroxy-ß-methylbutyrate**

(71) Applicant: Abbott Laboratories, Inc., Abbott Park, IL 60064 (US)
(72) Inventor: Ramírez, María, 18003 GRANADA (ES); Barranco-Pérez, Alejandro, 18110 LAS GABIAS (Granada) (ES); Rueda Cabrera, Ricardo, 18008 GRANADA (ES); Bespalov, Anton, 69118 HEIDELBERG (DE); Behl, Berthold, 67098 BAD DUERKHEIM (DE); Van Gaalen, Marcel M., 67433 NEUSTADT AN DER WEINSTRASSE (DE)
(74) Representative: Ungria López, Javier

(57) **Abstract**

A method of treating or preventing depression using β-hydroxy-β-methylbutyrate (HMB) is described. The method includes administering an effective amount of HMB or a pharmaceutically acceptable salt thereof to a subject in need thereof. The HMB can be administered as part of a nutritional composition. Also provided is a method of treating or preventing a condition which can be improved or prevented by the activation of mTOR signaling by administering an effective amount of HMB or a pharmaceutically acceptable salt thereof to a subject in need thereof.

## Description

### BACKGROUND

Major depression is the leading psychological disorder in the western world. It is growing in all age groups, in virtually every community, and the growth in rates of depression is seen most in the young, especially teens. At the rate of increase, it will be the 2nd most disabling condition in the world by 2020, behind heart disease. However, existing antidepressants such as serotonin uptake inhibitors generally require a prescription to obtain, which can deter those having depression from obtaining the help they need. Accordingly, there is a need for additional antidepressants, and in particular antidepressants that can be provided as a supplement without a prescription.

### SUMMARY

The inventors have discovered a method of treating or preventing depression that includes administering an effective amount of β-hydroxy-β-methylbutyrate (HMB) or a pharmaceutically acceptable salt thereof to a subject in need thereof The HMB is administered orally, and/or as part of a nutritional composition. In addition to HMB, an additional antidepressant can also be administered to the subjects. In some embodiments, the subject is a human, such as an elderly human.

In further embodiments, the nutritional composition including HMB contains 150-500 calories per serving and is in the form of a powder suitable for reconstitution to a liquid, a liquid or a bar. In additional embodiments, the nutritional composition includes at least one source of carbohydrate and at least one source of protein, which in other embodiments the nutritional composition includes at least one source of fat, at least one source of carbohydrate, and at least one source of protein.

In another aspect, the use of β-hydroxy-β-methylbutyrate (HMB) for the preparation of a medicament for treating a subject suffering from a condition which can be improved or prevented by the activation of mTOR signaling is provided. In further embodiments, the activation of mTOR signaling can occur in neuronal cells, and/or the condition from which the subject is suffering can be depression.

### BRIEF DESCRIPTION OF THE FIGURES

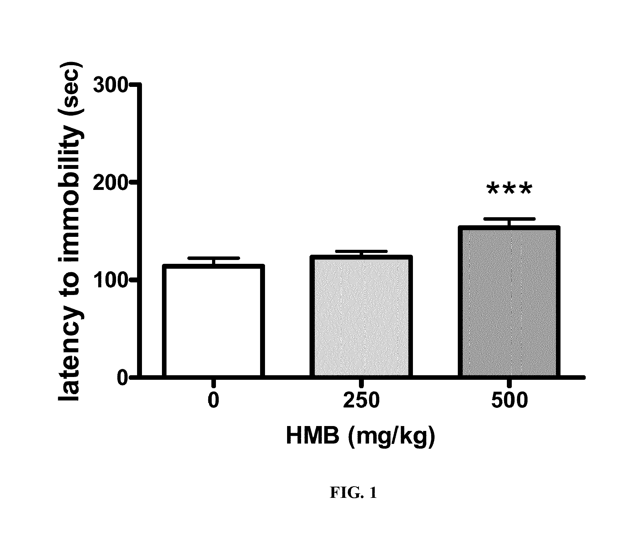

Figure 1 provides a bar graph comparing the amount of HMB administered to the latency to immobility seen in a swim test of rats (Forced Swim Test).

### DETAILED DESCRIPTION

Unless otherwise defined, all technical and scientific terms used herein have the same meaning as commonly understood by one of ordinary skill in the art to which this application pertains. Although methods and materials similar or equivalent to those described herein can be used in the practice or testing of the exemplary embodiments, suitable methods and materials are described below. In case of conflict, the present specification, including definitions, will control. In addition, the materials, methods, and examples are illustrative only and not intended to be limiting.

### Definitions

The terminology as set forth herein is for description of the embodiments only and should not be construed as limiting the application as a whole. Unless otherwise specified, "a," "an," "the," and "at least one" are used interchangeably. Furthermore, as used in the description of the application and the appended claims, the singular forms "a", "an", and "the" are inclusive of their plural forms, unless contraindicated by the context surrounding such.

The recitations of numerical ranges by endpoints include all numbers subsumed within that range *(e.g.,* 1 to 5 includes 1, 1.5, 2, 2.75, 3, 3.80, 4, 5, *etc.*)*.*

The expression "effective amount" as used herein, refers to a sufficient amount of agent to treat or prevent the development of depression, and to thereby exhibit a therapeutic effect. The exact amount required will vary from subject to subject, depending on the species, age, and general condition of the subject, the particular therapeutic agent, its mode and/or route of administration, and the like.

The term "elderly," as used herein, refers to an individual of at least 45 years of age, including at least 50 years of age, at least 55 years of age, at least 60 years of age, at least 65 years of age, at least 70 years of age, at least 75 years of age, and including at least 80 years of age or greater. The term "elderly" also includes the groups of from about 45 years of age to about 95 years of age, and the group of from about 55 years of age to about 80 years of age.

The subject is preferably a mammal, such as a human, a domesticated farm animal *(e.g.,* cow, horse, pig) or pet *(e.g.,* dog, cat). More preferably, the subject is a human. Subjects can be selected from various age groups. For example, subjects can be children, adults, or the elderly. Adult humans are subjects having an age of 18 or more, while human children have an age of 17 or less. Human subjects can be selected from any age group. For example subjects can have an age from 1 to 100, or any ages there between.

Depression is a decrease in mood that persists for an extended period of time. Major depressive disorder, or major depression, is characterized by a combination of symptoms that interfere with a person's ability to work, sleep, study, eat, and enjoy once-pleasurable activities. Major depression is disabling and prevents a person from functioning normally. Some people may experience only a single episode within their lifetime, but more often a person may have multiple episodes. Minor depression is characterized by having symptoms for 2 weeks or longer that do not meet full criteria for major depression. Without treatment, people with minor depression are at high risk for developing major depressive disorder.

Signs and symptoms of depression include persistent sad, anxious, or "empty" feelings, feelings of hopelessness or pessimism, feelings of guilt, worthlessness, or helplessness, irritability, restlessness, loss of interest in activities or hobbies once pleasurable, including sex, fatigue and decreased energy, difficulty concentrating, remembering details, and making decisions, insomnia, early-morning wakefulness or excessive sleeping, overeating or appetite loss, and thoughts of suicide, suicide attempts. People with depression may exhibit one or more of these symptoms, and do not all experience the same symptoms, or display symptoms with the same degree of severity.

Depression is generally caused by a combination of genetic, biological, environmental, and psychological factors. Longstanding theories about depression suggest that important neurotransmitters such as serotonin are out of balance in depression. Brain-imaging technologies, such as magnetic resonance imaging, have shown that the brains of people who have depression look different than those of people without depression. More specifically, the parts of the brain involved in mood, thinking, sleep, appetite, and behavior appear different. Genetics research indicates that risk for depression results from the influence of several genes acting together with environmental or other factors. In addition, trauma, loss of a loved one, a difficult relationship, or any stressful situation may trigger depression.

While not intending to be bound by theory, it is believed that HMB activates the mTOR signaling cascade, which is important in brain functionality and cognition. The mammalian target of rapamycin (mTOR) is an atypical serine/threonine kinase that is present in two distinct complexes. mTOR complex 1 (mTORC1) is composed of mTOR, Raptor, GβL (mLST8), and Deptor and is partially inhibited by rapamycin. mTORC1 integrates multiple signals reflecting the availability of growth factors, nutrients, or energy to promote either cellular growth when conditions are favorable or catabolic processes during stress or when conditions are unfavorable. See Hoeffer et al., Trends Neurosci. 33(2), p. 67-75 (2010). In one embodiment, the activation of mTOR signaling by HMB occurs in neuronal cells. In a further embodiment, the subject being administered HMB in order to activate mTOR signaling is suffering from the condition of depression.

A method of treating or preventing depression is provided herein that includes administering an effective amount of β-hydroxy-β-methylbutyrate (HMB) or a pharmaceutically acceptable salt thereof to a subject in need thereof. A subject in need of treatment of depression is a subject who currently has depression. The depression can be either minor depression or major depression. Treatment of the depression preferably results in a decrease or the elimination of one or more of the symptoms of depression. An example of a subject in need of treatment of depression is a subject who has been diagnosed as having depression.

A subject in need of preventing depression is a subject who is at an increased risk of having depression. Examples of subjects who are at an increased risk of having depression include subjects who have previously had depression, in which case the method of preventing depression would represent a method of preventing a relapse of depression. Other examples of subjects in need of preventing depression are subjects who have any of the risk factors known to be associated with the development of depression, such as bereavement, a family history of depression, prolonged inactivity, or various forms of unhealthy stress.

Treatment or prevention of depression is particularly useful for human subjects. However, research has established that animals can suffer from depression, and thus treatment or prevention of depression in animals may also be useful. Animals who are depressed appear to experience a profound feeling of sadness, similar to that felt by humans who are depressed. For example, domesticated animals such as dogs and horses are known to be susceptible to depression. Nonetheless, due to the highly complex nature of the human mind, humans are particularly prone to the development of depression, and the development of depression in humans can represent a particularly important problem to treat and prevent.

Administration of HMB can also be used to treat or prevent depression in elderly humans. Depression is not a normal part of aging. However, when older adults do have depression, it may be overlooked because seniors may show different, less obvious symptoms. Older adults also may have more medical conditions such as heart disease, stroke, or cancer, which may cause depressive symptoms. In addition, elderly humans may be taking medications with side effects that contribute to depression. In particular, elderly humans who are inactive as a result of disease, medical treatment, or sarcopenia may be prone to depression. Although it is typically assumed that young people exhibit the highest rates of suicide due to depression, older white males age 85 and older actually have the highest suicide rate in the United States. Accordingly, it can be of particular importance to treat or prevent depression in elderly human subjects.

An additional risk factor for developing depression that is particularly likely to be an issue for elderly patients is sarcopenia. Sarcopenia is a degenerative loss of skeletal muscle mass and strength that is generally associated with aging, but can also be caused by a significant loss of opportunity for exercise, as can occur as a result of immobility due to prolonged bed rest. Sarcopenia differs from regular muscle atrophy, and involves a loss of lean body mass and replacement of that body mass with fat. While sarcopenia is generally seen as one of the effects of aging, it can exist to substantially different degrees in individuals based on genetic and environmental factors, such as the extent to which an individual exercises.

The method of treating or preventing depression by administering HMB to a subject can also include administration of an additional antidepressant compound. As discussed herein, additional antidepressant compounds generally fall into two categories, depression-treating drugs and depression-treating non-drugs. As used herein, the term depression-treating drugs should be understood to refer to compounds that are either now known or are discovered in the future to be useful and effective in treating depression and that can only be administered or consumed under the supervision of a medical professional (e.g., a prescription drug). As used herein, the term depression-treating non-drugs should be understood to refer to compounds that are either now known or are discovered in the future to be useful and effective in treating depression but that do not require administration or consumption be limited to being under the supervision of a medical professional (e.g., an over-the-counter or OTC drug). Antidepressant compounds include the older antidepressants such as tricyclic compounds and monoamine oxidase inhibitors. Newer antidepressant compounds include selective serotonin reuptake inhibitors (SSRIs) such as fluoxetine (Prozac), sertraline (Zoloft), escitalopram (Lexapro), paroxetine (Paxil), and citalopram (Celexa) and serotonin and norepinephrine reuptake inhibitors (SNRIs), which are similar to SSRIs and include venlafaxine (Effexor) and duloxetine (Cymbalta). Antidepressants also include (non-prescription) botanical/natural supplements such as extract of St. John's wort (Hypericum perforatum), which is used extensively in Europe to treat mild to moderate depression, saffron, rhodiola rosea, lavender, and echium. In certain embodiments of the methods that are disclosed herein, the effective amount of HMB is administered along with one or more depression-treating drugs, along with one or more depression-treating non-drugs, or along with one or more depression-treating drugs or depression-treating non-drugs. In certain embodiments of the methods that are disclosed herein, one or more depression-treating drugs is included in the nutritional composition along with the effective amount of HMB. The depression treating drugs may be selected from the group consisting of tricyclic compounds, monoamine oxidase inhibitors, selective serotonin reuptake inhibitors (SSRIs) *(e.g.,* fluoxetine (Prozac), sertraline (Zoloft), escitalopram (Lexapro), paroxetine (Paxil), and citalopram (Celexa)), serotonin and norepinephrine reuptake inhibitors (SNRIs) (*e.g*., venlafaxine (Effexor) and duloxetine (Cymbalta), and combinations thereof. In other embodiments of the methods that are disclosed herein, one or more depression-treating non-drugs is included in the nutritional composition along with the effective amount of HMB. The depression-treating non-drugs may be selected from the group consisting of St. John's wort,. When the effective amount of HMB is provided in a nutritional composition, it may be advantageous from a product availability standpoint to limit any additional depression-treating compounds to non-drugs so that the nutritional composition is more readily available to the consumer.

In some embodiments, it can be helpful to supplement HMB administration with exercise. Preferably, the exercise is part of a program of regular exercise by the subject. Exercise includes flexibility exercises, such as stretching, which improve the range of motion of muscles and joints; aerobic exercises, such as cycling, swimming, walking, skipping rope, rowing, running, hiking or playing tennis, which focus on increasing cardiovascular endurance; and anaerobic exercises, such as weight training, functional training, eccentric training or sprinting and high-intensity interval training, which increase short-term muscle strength.

In addition to depression, in certain embodiments disclosed herein, the effective amount of HMB can be administered to treat or prevent depression-related disorders. (The exemplary effective amounts and other formulation-related discussion is provided herein with respect to using HMB treating or preventing depression should be considered equally applicable to the treatment or prevention of depression-related disorders.) Examples of depression-related disorders suitable for treatment with an effective amount of HMB include bipolar disorder, acute and chronic stress-related disorders including generalized anxiety, post-traumatic stress disorder, phobias, panic disorder, burnout syndrome, jet-lag syndrome, and social anxiety), age-associated mood disturbances and disorders, menopause-associated mood disturbances and disorders, and chronic pain syndrome are other examples of depression-related disorders. (Notably, the age and menopause-associated mood disturbances and disorders may present alone or in the context of another disease. Moreover, the phrase acute and chronic stress-related disorders should be considered to include conditions associated with sustained stress exposure, including, but not limited to, extended working hours, working night shifts, working irregular shifts and frequent changes of time zones.) Depression-related disorders frequently involve diminished mTOR signaling in neural cells. Accordingly, in certain embodiments of the methods described herein, an effective amount of HMB is used to treat a subject suffering from a condition which can be improved or prevented by the activation of mTOR signaling, by administering the effective amount of β-hydroxy-β-methylbutyrate (HMB) to a subject in need thereof.

The method of treating or preventing depression includes administering an effective amount of β-hydroxy-β-methylbutyrate (HMB). β-hydroxy-β-methylbutyrate is a metabolite of the essential amino acid leucine, and has the IUPAC name 3-hydroxy-3-methylbutanoic acid. A preferred form of HMB is the calcium salt of HMB, also designated as Ca-HMB, which is most typically the monohydrate calcium salt. The HMB used can come from any source. Calcium HMB monohydrate is commercially available from Technical Sourcing International (TSI) of Salt Lake City, Utah. Note that all amounts of HMB described herein are based on use of HMB in free form, although those skilled in the art will understand that when the HMB is administered via a different compound, relatively more or less that the compound may be required to provide the same amount of HMB, depending upon the molecular weight of the particular compound.

The term "HMB" as used herein is intended to include both the free acid and salt forms of β-hydroxy-β-methylbutyrate. (Notably, in the claims the phrase HMB or a pharmaceutically acceptable salt thereof has been used to avoid any doubt that pharmaceutically acceptable salts should be included.) Although the calcium monohydrate salt is a form of HMB specifically contemplated for use in certain embodiments of the methods describe herein, other suitable sources include HMB as a free acid, a salt, an anhydrous salt, an ester, a lactone, or other product forms that provide a bioavailable form of HMB suitable for administration. Non-limiting examples of suitable salts of HMB (hydrated or anhydrous) for use herein include sodium, potassium, chromium, calcium, and other non-toxic salt forms.

### HMB Formulations

The HMB can be formulated in a suitable composition and then, in accordance with the methods described herein, administered to a subject in a form adapted to the chosen route of administration. The formulations include, but are not limited to, those suitable for oral or parental (including subcutaneous, intramuscular, intraperitoneal, intratumoral, and intravenous) administration. Oral administration, as defined herein, includes any form of administration in which the HMB passes through the esophagus of the subject. For example, oral administration includes nasogastric intubation, in which a tube is run from through the nose to the stomach of the subject to administer food or drugs.

Pharmaceutical and nutritional formulations including HMB can also be referred to herein as medicaments. For example, HMB can be used for the preparation of a medicament for treating a subject suffering from a condition which can be improved or prevented by the activation of mTOR signaling.

Compositions including HMB, such as nutritional compositions, can be provided to a subject in one or more servings over a period of time. When a nutritional composition is administered in more than one serving, the nutritional composition is divided into separate portions which are provided to the subject at different points in time. Thus, when more than one serving of a composition is provided to a subject, reference to the amounts of ingredients present in the composition refer to those present before the composition is divided, unless specified otherwise. Typically, if more than one serving is taken from a composition, they are administered over the course of a single day. The term "serving" as used herein, unless otherwise specified, is intended to be construed as any amount which is intended to be consumed by an individual in one sitting or within one hour or less.

HMB can be administered to a subject one or more times per day for a period suitable to achieve the desired effect. For example, HMB can be administered to a subject every day for at least a week, every day for at least two weeks, every day for at least a month, every day for at least 6 months, or every day for a year or more. Within the context of providing a dose to a subject, every day is intended to reflect a subject who has been instructed to be administered HMB every day, and who actually is administered HMB for at least 90% of the days during the desired period of administration.

Formulations include, but are not limited to, those suitable for oral or parental (including subcutaneous, intramuscular, intraperitoneal, intratumoral, and intravenous) administration. Oral administration, as defined herein, includes any form of administration in which the HMB passes through the esophagus of the subject. For example, oral administration includes nasogastric intubation, in which a tube is run from through the nose to the stomach of the subject to administer food or drugs.

Oral formulations include liquids, powders, semi-solids, semi-liquids compositions, provided that such a formulation allows for the safe and effective oral delivery of HMB and optional nutritive components. In certain embodiments, the oral formulation is a nutritional composition. A nutritional composition is a composition that is edible and includes additional nutrients beyond HMB, such as vitamins, carbohydrates, fats, and proteins. Formulations suitable for oral administration may be presented as discrete units such as tablets, troches, capsules, lozenges, wafers, or cachets, each containing a predetermined amount of the HMB as a powder or granules or as a solution or suspension in an aqueous liquor or non-aqueous liquid such as a syrup, an elixir, an emulsion, or a draught.

The term "nutritional liquid" as used herein, unless otherwise specified, refers to nutritional compositions in ready-to-drink liquid form, concentrated form, and nutritional liquids made by reconstituting the nutritional powders described herein prior to use. The nutritional liquid may also be formulated as a suspension, an emulsion, a solution, and so forth.

The term "nutritional powder" or "reconstitutable powder" as used herein, unless otherwise specified, refers to nutritional compositions in flowable or scoopable form that can be reconstituted with water or another aqueous liquid prior to consumption and includes both spray dried and drymixed/dryblended powders.

The term "nutritional semi-solid," as used herein, unless otherwise specified, refers to nutritional products that are intermediate in properties, such as rigidity, between solids and liquids. Some semi-solids examples include puddings, yogurts, gels, gelatins, and doughs.

The term "nutritional semi-liquid," as used herein, unless otherwise specified, refers to nutritional compositions that are intermediate in properties, such as flow properties, between liquids and solids. Some semi-liquids examples include thick shakes, liquid yogurts, and liquid gels.

The concentration of HMB in the nutritional composition may range up to about 10%, including from about 0.01% to about 10%, also including from about 0.1 % to about 5.0%, also including from about 0.3 to about 4%, also including from about 0.5% to about 2%, and also including from about 0.4% to about 1.5% by weight of the nutritional liquid composition. While HMB can be provided in a variety of percent amounts within a nutritional composition, it should be understood that the overall amounts are further limited by the gram amounts described below, such that a relatively large concentration will generally have a lower concentration, in order to avoid providing an excessive amount of HMB.

The nutritional composition provided to the subjects can provide from 0.1 g/day to 10 g/day of HMB. Alternately, the composition can provide 0.5 g/day to 10 g/day of HMB, 1.5 g/day to 10 g/day, 0.5 g/day to 5 g/day, or 1.5 g/day to 4 g/day. Subjects may be administered one serving per day, two servings per day, three servings per day, or four or more servings per day to receive the desired amount of HMB from the nutritional composition. In some embodiments, each serving of a nutritional composition including HMB provides about 1.5 grams of HMB. In addition, in further embodiments, the HMB can be provided to the subject for at least about a week.

In certain embodiments, the HMB is provided to the subject as part of a nutritional composition. The nutritional composition includes one or more ingredients that help satisfy the subject's nutritional requirements, in addition to providing a useful formulation for the HMB. For example, the nutritional composition can include a fat, a carbohydrate, a protein and combinations thereof. In certain embodiments, the nutritional composition includes at least one source of fat, at least one source of carbohydrate, and at least one source of protein. In some embodiments, the nutritional composition can be formulated to provide a specialized nutritional product for use in subjects afflicted with specific diseases or conditions. Many different sources and types of proteins, fats, and carbohydrates are known and can be used in nutritional compositions that include HMB. In certain embodiments, the nutritional composition is in the form of a powder suitable for reconstitution to a liquid, a ready-to-drink liquid or a bar.

In certain embodiments, the nutritional composition may be a solid nutritional product. Non-limiting examples of solid nutritional products include snack and meal replacement products, including those formulated as bars, sticks, cookies or breads or cakes or other baked goods, frozen liquids, candy, breakfast cereals, powders or granulated solids or other particulates, snack chips or bites, frozen or retorted entrees and so forth. In certain embodiments, when the nutritional composition is a solid product, the serving may be 25 grams to 150 grams.

In certain embodiments, the nutritional composition may be a nutritional liquid. Non-limiting examples of nutritional liquids include snack and meal replacement products, hot or cold beverages, carbonated or non carbonated beverages, juices or other acidified beverages, milk or soy-based beverages, shakes, coffees, teas, enteral feeding compositions, and so forth. Generally, the nutritional liquids are formulated as suspensions or emulsions, but the nutritional liquids can also be formulated in any other suitable forms such as clear liquids, solutions, liquid gels, liquid yogurts, and so forth. In certain embodiments, when the nutritional composition is a liquid nutritional product, the serving may be 150 milliliters to 500 milliliters. In certain other embodiments, when the nutritional composition is a liquid, the serving is 237 milliliters (~8 fl. oz.). In other embodiments, when the nutritional composition is a liquid, the serving is 177 milliliters to 296 milliliters (~6 fl. oz. to ~10 fl. oz.). In yet other embodiments, when the nutritional composition is a liquid, the serving is 207 milliliters to 266 milliliters (~7 fl. oz. to ~9 fl. oz.).

In yet other embodiments, the nutritional composition may be formulated as semi-solid or semi-liquid compositions (e.g., puddings, gels, yogurts, etc.), as well as more conventional product forms such as capsules, tablets, caplets, pills, and so forth. In other embodiments, the nutritional composition may be in the form of lozenges, tablets (e.g., chewable, coated, etc.), pastes, gels, or yogurts.

Examples of nutritional composition forms suitable for use herein include snack and meal replacement products, including those formulated as bars, sticks, cookies or breads or cakes or other baked goods, frozen liquids, candy, breakfast cereals, powders or granulated solids or other particulates, snack chips or bites, frozen or retorted entrees, and so forth. The nutritional composition can also be forms that fall between solid and liquid, such as semi-solid or semi-liquid compositions such as puddings or gels.

Examples of liquid product forms suitable for use herein include snack and meal replacement products, hot or cold beverages, carbonated or non-carbonated beverages, juices or other acidified beverages, milk or soy-based beverages, shakes, coffees, teas, compositions for administration by nasogastric intubation, and so forth. These liquid compositions are most typical formulated as suspensions or emulsions, but can also be formulated in any other suitable form such as clear liquids, substantially clear liquids, liquid gels, and so forth.

The nutritional composition includes one or more ingredients that help satisfy the subjects' nutritional requirements. The optional nutrients can provide up to about 1000 kcal of energy per serving or dose, including from about 25 to about 900 kcal, from about 75 to about 700 kcal, from about 150 to about 500 kcal, from about 350 to about 500 kcal, or from about 200 to about 300 kcal per serving.

In certain embodiments, the nutritional composition may comprise 8 grams to 100 grams of protein per serving or 10 grams to 100 grams of protein per serving. In other embodiments, the nutritional composition may comprise 10 grams to 50 grams of protein per serving. In still other embodiments, the nutritional composition may comprise 10 grams to 25 grams of protein per serving. Virtually any source of protein may be used so long as it is suitable for use in oral nutritional compositions and is otherwise compatible with any other selected ingredients or features in the nutritional composition.

The source of protein may include, but is not limited to, intact, hydrolyzed, and partially hydrolyzed protein, which may be derived from any known or otherwise suitable source such as milk (*e.g*., casein, whey), animal (*e.g*., meat, fish), cereal *(e.g.,* rice, corn), vegetable (*e.g*., soy, pea), and combinations thereof. Non-limiting examples of the source of protein include milk protein isolates, milk protein concentrates, casein protein isolates, whey protein concentrates, whey protein isolates, whey protein hydrolysates, sodium or calcium caseinates, whole cow's milk, partially or completely defatted milk, soy protein isolates, soy protein concentrates, soy protein hydrolysates, pea protein concentrates, pea protein isolates, pea protein hydrolysates, and so forth. In addition, the nutritional composition may include at least 8 grams of protein per serving, and can comprise any one source of protein or any combination of any of the various sources of protein listed above.

In certain embodiments, the nutritional composition may include at least one source of fat. In other embodiments, the nutritional composition may include no fat, or essentially no fat *(i.e.,* less than 0.5 grams of fat per serving). In some embodiments where the nutritional composition contains fat, the nutritional composition may comprise 2 grams to 45 grams of at least one source of fat per serving. In other embodiments, the nutritional composition may comprise 5 grams to 35 grams of at least one source of fat per serving. In yet other embodiments, the nutritional composition may comprise 15 grams to 30 grams of at least one source of fat per serving.

In general, any source of fat may be used so long as it is suitable for use in oral nutritional compositions and is otherwise compatible with any other selected ingredients or features present in the nutritional composition. The source of fat may be derived from plants, animals, and combinations thereof. Non-limiting examples of suitable sources of fat for use in the nutritional compositions described herein include coconut oil, fractionated coconut oil, soy oil, corn oil, olive oil, safflower oil, high oleic safflower oil, MCT oil (medium chain triglycerides), sunflower oil, high oleic sunflower oil, palm and palm kernel oils, palm olein, canola oil, marine oils, cottonseed oils, and combinations thereof

In certain embodiments, the nutritional composition may include at least one source of carbohydrate. In some embodiments, the nutritional composition may comprise 15 grams to 110 grams of at least one source of carbohydrate per serving. In other embodiments, the nutritional composition may comprise 25 grams to 90 grams of at least one source of carbohydrate per serving. In yet other embodiments, the nutritional composition may comprise 40 grams to 65 grams of at least one source of carbohydrate per serving.

The at least one source of carbohydrate suitable for use in the nutritional compositions disclosed herein may be simple, complex, or variations or combinations thereof. Generally, any source of carbohydrate may be used so long as it is suitable for use in oral nutritional compositions and is otherwise compatible with any other selected ingredients or features present in the nutritional composition. Non-limiting examples of a source of carbohydrate suitable for use in the nutritional emulsions described herein may include maltodextrin, hydrolyzed or modified starch or cornstarch, glucose polymers, corn syrup, corn syrup solids, rice-derived carbohydrates, sucrose, glucose, fructose, lactose, high fructose corn syrup, honey, sugar alcohols (*e.g*., maltitol, erythritol, sorbitol), and combinations thereof.

The amount or concentration of the at least one source of fat, at least one source of protein, and at least one source of carbohydrate present in certain embodiments of the nutritional compositions described herein may vary widely depending on the product formulation of the nutritional composition (*e.g.*, solid product, liquid, etc.). In addition, the amount or concentration of the at least one source of fat, at least one source of protein, and at least one source of carbohydrate present in certain embodiments of the nutritional compositions described herein may be characterized based upon: (i) a percentage of the total calories per serving in the nutritional composition; or (ii) the total weight of each ingredient present in a serving of the nutritional composition; or both (i) and (ii). For example, in certain embodiments, the amount or concentration of the at least one source of fat, at least one source of protein, and the at least one source of carbohydrate present in the nutritional composition can be within the ranges shown in the examples provided in Tables I and II below. As previously discussed, in other embodiments, the nutritional composition contains no fat or essentially no fat (*i.e*., less than 0.5 grams per serving).

**Table I**

| Nutrient (% total calories) | Example A | Example B | Example C |
|---|---|---|---|
| Carbohydrate | 0-94 | 10-85 | 20-60 |
| Fat | 0-94 | 5-80 | 20-60 |
| Protein | 6-100 | 10-85 | 20-60 |

**Table II**

| Nutrient (grams per serving) | Example D | Example E | Example F |
|---|---|---|---|
| Carbohydrate | 15-112 | 25-90 | 40-65 |
| Fat | 2-45 | 5-35 | 15-30 |
| Protein | 8-100 | 10-50 | 20-30 |

In certain embodiments, the nutritional composition comprises at least one source of fat and at least one source of carbohydrate, and the at least one source of fat provides 5 percent to 80 percent of the caloric density per serving and the at least one source of carbohydrate provides 10 percent to 85 percent of the caloric density per serving. In other embodiments, the nutritional composition comprises at least one source of fat and at least one source of carbohydrate, and the at least one source of fat provides 10 percent to 65 percent of the caloric density per serving and the at least one source of carbohydrate provides 20 percent to 75 percent of the caloric density per serving. In yet other embodiments, the nutritional composition comprises at least one source of fat and at least one source of carbohydrate, and the at least one source of fat provides 30 percent to 50 percent of the caloric density per serving and the at least one source of carbohydrate provides 30 percent to 50 percent of the caloric density per serving. Such embodiments provide flexibility in formulating calorie dense nutritional compositions with various other ingredients.

The HMB-containing nutritional composition can also include other ingredients that may modify the physical, nutritional, chemical, hedonic, or processing characteristics of the product or serve as pharmaceutical or additional nutritional components. Non-limiting examples of such optional ingredients include preservatives, antioxidants, emulsifying agents, buffers, fructooligosaccharides, chromium picolinate, pharmaceutical additives, colorants, flavors or masking agents, thickening agents and stabilizers, artificial sweeteners, hydrocolloids such as guar gum, xanthan gum, carrageenan, gellan gum, gum acacia, and so forth.

The HMB-containing nutritional composition can also include vitamins, minerals, and combinations thereof. Exemplary vitamins include, but are not limited to, vitamin A, vitamin E, vitamin D2, vitamin D3, vitamin A palmitate, vitamin E acetate, vitamin C palmitate (ascorbyl palmitate), vitamin K, thiamine, riboflavin, pyridoxine, vitamin B12, carotenoids (*e.g*., beta-carotene, zeaxanthin, lutein, lycopene), niacin, folic acid, pantothenic acid, biotin, vitamin C, choline, inositol, salts and derivatives thereof, and combinations thereof. Exemplary minerals include, but are not limited to, calcium, selenium, potassium, iodine, phosphorus, magnesium, iron, zinc, manganese, copper, sodium, molybdenum, chromium, chloride, and combinations thereof.

An exemplary nutritional composition suitable for formulating HMB is described in Table III below, with the specific ingredients provided immediately thereafter.

**Table III: Liquid Formulation Information**

| | UNIT | per 8 fl oz |
|---|---|---|
| Energy EU | kcal | 350 |
| Protein | g | 20 |
| Fat | g | 11 |
| Linoleic acid | g | 3 |
| Carbohydrate | g | 44 |
| Fructooligosaccharide | g | 3 |
| Sugar | g | 20 |
| Ca-HMB | g | 1.5 |
| VITAMINS | | |
| Vitamin A (Palmitate) | IU | 1000 |
| Vitamin A (B-Carotene) | IU | 0 |
| Vitamin D₃ | IU | 160 |
| Vitamin E | IU | 30 |
| Vitamin K₁ | mcg | 20 |
| Vitamin C | mg | 60 |
| Folic Acid | mcg | 200 |
| Vitamin B₁ | mg | 0.38 |
| Vitamin B₂ | mg | 0.43 |
| Vitamin B₆ | mg | 0.5 |
| Vitamin B₁₂ | mcg | 3 |
| Niacin | mg | 5 |
| Pantothenate | mg | 2.5 |
| Biotin | mcg | 75 |
| L-carnitine | mg | 43 |
| Choline | mg | 83 |
| MINERALS | | |
| Sodium | mg | 240 |
| Potassium | mg | 560 |
| Chloride | mg | 150 |
| Calcium | mg | 500 |
| Phosphorus | mg | 350 |
| Magnesium | mg | 100 |
| Iron | mg | 4.5 |
| Zinc | mg | 15 |
| Manganese | mg | 0.50 |
| Copper | mg | 0.50 |
| Iodine | mcg | 25 |
| Selenium | mcg | 30 |
| Chromium | mcg | 30 |
| Molybdenum | mcg | 30 |

The nutritional composition described in Table III includes Water, Corn syrup, Sucrose, Milk Protein Concentrate, Sodium Caseinate, Canola Oil, Corn Oil, Fructooligosaccharides, Soy Protein Isolate, Calcium Beta-Hydroxy-Beta-Methylbutyrate, Whey Protein Concentrate, Potassium Citrate, Natural and Artificial Flavors, Potassium Phosphate, Lecithin, Cellulose Gel, Magnesium Hydroxide, Calcium Carbonate, Ascorbic Acid, Calcium Phosphate, Choline Chloride, Sodium Chloride, Sodium Phosphate, Potassium Hydroxide, Zinc Sulfate, Cellulose Gum, L-Carnitine, Carrageenan, dl-Alpha-Tocopherol Acetate, Dextrose, Ferrous Sulfate, Maltodextrin, Niacinamide, Gellan Gum, Calcium Pantothenate, Citric Acid, Cupric Sulfate, Manganese Sulfate, Chromium Chloride, Thiamine Chloride Hydrochloride, Coconut Oil, Vitamin A Palmitate Pyridoxine Hydrochloride, Riboflavin, Folic Acid, Biotin, Sodium Selenate, Sodium Molybdate, Potassium Iodide, Phylloquinone, Cyanocobalamin, and Vitamin D3.

An exemplary liquid nutritional composition formulated for use with diabetic subjects suitable for formulating HMB is provided in Table IV below, with the specific ingredients provided immediately thereafter.

**Table IV: Diabetic Formulation Information**

| | UNIT | per 8 fl oz |
|---|---|---|
| Nutrient Density | Cal/ml | 0.93 |
| Protein | % Cal | 18 |
| Carbohydrate | % Cal | 47 |
| Fat | % Cal | 35 |
| Osmolality | mOsm/L | 86 |
| Viscosity | | Thin |
| Protein | g | 9.9 |
| Carbohydrate | g | 29.3 |
| Fat | g | 8.6 |
| Water | g | 200 |
| Ca-HMB | g | 1.5 |
| VITAMINS | | |
| Vitamin A (Palmitate) | IU | 1750 |
| Vitamin A (B-Carotene) | mg | 0.5 |
| Vitamin D | IU | 100 |
| Vitamin E | IU | 30 |
| Vitamin K | mcg | 20 |
| Vitamin C | mg | 60 |
| Folic Acid | mcg | 200 |
| Vitamin B₁ | mg | 0.38 |
| Vitamin B₂ | mg | 0.43 |
| Vitamin B₆ | mg | 1.0 |
| Vitamin B₁₂ | mcg | 3.0 |
| Niacin | mg | 5.0 |
| Pantothenate | mg | 2.5 |
| Biotin | mcg | 75 |
| L-carnitine | mg | 43 |
| Choline | mg | 100 |

| MINERALS | | |
|---|---|---|
| Sodium | mg | 210 |
| Potassium | mg | 370 |
| Chloride | mg | 355 |
| Calcium | mg | 250 |
| Phosphorus | mg | 250 |
| Magnesium | mg | 100 |
| Iron | mg | 4.5 |
| Zinc | mg | 3.8 |
| Manganese | mg | 1.0 |
| Copper | mg | 0.50 |
| Iodine | mcg | 38 |
| Selenium | mcg | 18 |
| Chromium | mcg | 120 |
| Molybdenum | mcg | 38 |

The liquid diabetic nutritional composition described in Table IV includes Water, Corn Maltodextrin, Sodium & Calcium Caseinates, Maltitol Syrup, High Oleic Safflower Oil, Fructose, Soy Protein Isolate, Soy Fiber, Short-Chain Fructooligosaccharides, Canola Oil, Calcium Phosphate, Magnesium Chloride, Soy Lecithin, Artificial Flavor, Sodium Citrate, Magnesium Phosphate, Potassium Citrate, Potassium Chloride, Potassium Phosphate, Ascorbic Acid, Choline Chloride, dl-Alpha- Tocopheryl Acetate, Gellan Gum, Acesulfame Potassium, Ferrous Sulfate, Zinc Sulfate, Niacinamide, Manganese Sulfate, Calcium Pantothenate, Cupric Sulfate, Sucralose, Pyridoxine Hydrochloride, Thiamine Chloride Hydrochloride, Vitamin A Palmitate, Riboflavin, Chromium Chloride, Beta-Carotene, Folic Acid, Biotin, Sodium Molybdate, Potassium Iodide, Sodium Selenate, Phylloquinone, Cyanocobalamin, and Vitamin D3.

An exemplary liquid nutritional composition containing essentially no fat, having a clear, juice-like appearance and an acidic pH suitable for formulating HMB is provided in Table V below, with the specific ingredients provided immediately thereafter.

**Table V: Clear Formulation Information**

| | UNIT | per 10 fl oz |
|---|---|---|
| Nutrient Density | Cal/ml | 0.61 |
| Protein | % Cal | 20 |
| Carbohydrate | % Cal | 80 |
| Fat | % Cal | 0 |
| Osmolality | mOsm/L | |
| Viscosity | | Thin |
| Protein | g | 9 |
| Carbohydrate | g | 35 |
| Fat | g | 0 |
| | | |
| Ca-HMB | g | 1.5 |
| VITAMINS | | |
| | | |
| Vitamin A | % DV | 30 |
| Vitamin D | % DV | 15 |
| Vitamin E | % DV | 30 |
| Vitamin K | % DV | 30 |
| Vitamin C | % DV | 45 |
| Thiamin | % DV | 30 |
| Folic Acid | % DV | 15 |
| Riboflavin | % DV | 20 |
| Vitamin B₆ | % DV | 20 |
| Vitamin B₁₂ | % DV | 20 |
| Niacin | % DV | 10 |
| Pantothenic acid | % DV | 8 |
| Biotin | % DV | 10 |
| | | |
| MINERALS | | |
| Calcium | % DV | 6 |
| Phosphorus | % DV | 20 |
| Magnesium | % DV | 2 |
| Iron | % DV | 15 |
| Zinc | % DV | 30 |
| Manganese | % DV | 50 |
| Copper | % DV | 15 |
| Iodine | % DV | 35 |
| Selenium | % DV | 20 |
| Chromium | % DV | 15 |
| Molybdenum | % DV | 50 |

The liquid nutritional composition described in Table V includes Water, Corn Syrup Solids, Sugar, Whey Protein Isolate and less than 0.5% of the following: Citric Acid, Natural & Artificial Flavor, Phosphoric Acid, Ascorbic Acid, Acesulfame Potassium, Sucralose, Zinc Sulfate, dl-Alpha-Tocopheryl Acetate, Ferrous Sulfate, Niacinamide, Manganese Sulfate, Calcium Pantothenate, Cupric Sulfate, FD&C Yellow #6, Vitamin A Palmitate, Thiamine Chloride Hydrochloride, Pyridoxine Hydrochloride, FD&C Red #40, Riboflavin, Folic Acid, Chromium Chloride, Sodium Molybdate, Biotin, Potassium Iodide, Sodium Selenate, Phylloquinone, Vitamin D3, and Cyanocobalamin.

The various embodiments of the nutritional composition may be prepared by any process or suitable method (now known or known in the future) for making a selected product form, such as a nutritional solid, a nutritional powder, or a nutritional liquid. Many such techniques are known for any given product form such as nutritional liquids or nutritional powders and can easily be applied by one of ordinary skill in the art to the various embodiments of the nutritional composition according to the first, second, third, and fourth embodiments disclosed herein.

Liquid nutritional compositions can be manufactured by any process or suitable method for making nutritional emulsions. In one suitable manufacturing process, at least three separate slurries are prepared. These slurries include: a protein-in-fat (PIF) slurry, a carbohydrate-mineral (CHO-MIN) slurry and a protein-in-water (PIW) slurry. The PIF slurry is formed by heating and mixing any oils that are selected for the fat component (when present) and then adding an emulsifier *(e.g.,* lecithin), fat-soluble vitamins and a portion of the total protein (preferably about half of the milk protein concentrate) with continued heat and agitation. The CHO-MIN slurry is formed by adding to water (with heat and agitation), minerals *(e.g.,* potassium citrate, dipotassium phosphate, sodium citrate, *etc.),* trace and ultra trace minerals (often as pre-mix(es)), thickening-type or suspending agents *(e.g.,* Avicel, gellan, carragenan) and any HMB source. The CHO-MIN slurry that results is held for 10 minutes with continued heat and agitation and then additional minerals may be added (e.g., potassium chloride, magnesium carbonate, potassium iodide, *etc.)* and/or carbohydrates (e*.g.*, fructooligosaccharides, sucrose, corn syrup, *etc.).* The PIW slurry is formed by mixing the remaining protein (*i.e.*, sodium caseinate, soy protein, why protein, *etc.)* into water.

The three slurries are blended together with heat and agitation and the pH is adjusted to the desired range (typically near neutral, around 6.6-7), after which the composition is subjected to high-temperature short-time (HTST) processing during which time the composition is heat treated, emulsified and homogenized and allowed to cool. Water soluble vitamins and ascorbic acid are added (if applicable), the pH is again adjusted (if necessary), flavors are added and any additional water can be added to adjust the solids content to the desired range.

A nutritional solid, such as a spray dried nutritional powder or drymixed nutritional powder, may be prepared by any collection of known or otherwise effective technique, suitable for making and formulating a nutritional powder.

For example, when the nutritional powder is a spray dried nutritional powder, the spray drying step may likewise include any spray drying technique that is known for or otherwise suitable for use in the production of nutritional powders. Many different spray drying methods and techniques are known for use in the nutrition field, all of which are suitable for use in the manufacture of the spray dried nutritional powders herein.

One method of preparing the spray dried nutritional powder comprises forming and homogenizing an aqueous slurry or liquid comprising predigested fat, and optionally protein, carbohydrate, and other sources of fat, and then spray drying the slurry or liquid to produce a spray dried nutritional powder. The method may further comprise the step of spray drying, drymixing, or otherwise adding additional nutritional ingredients, including any one or more of the ingredients described herein, to the spray dried nutritional powder.

Other suitable methods for making nutritional products are described, for example, in U.S. Pat. No. 6,365,218 (Borschel, et al.*),* U.S. Pat. No. 6,589,576 (Borschel, et al.*),* U.S. Pat. No. 6,306,908 (Carlson, et al.)*,* U.S. Pat. Appl. No. 20030118703 A1 (Nguyen, et al.)*,* which descriptions are incorporated herein by reference to the extent that they are consistent herewith.

The following examples are included for purposes of illustration and are not intended to limit the scope of the invention.

### EXAMPLES

### Example 1: Evaluation of the Effect of HMB on Rat Performance in the Forced Swimming Test

The antidepressant effect of HMB was evaluated using the forced swimming test (FST) using the procedure described by Porsolt et al. (Eur. J. Pharmacol. 47(4). 379-91 (1978)). To begin the experiment, rats were individually placed inside vertical cylinders (height 40 cm; diameter 21.5 cm) containing 30 cm of water at 35 °C for 15 minutes (i.e., "pre-swim). Following this pre-swim period, the rats were removed and allowed to dry in a heated enclosure before returning to their cages. The rats were then orally administered a dose of either 250 mg/kg body weight or 500 mg/kg body weight HMB.

Twenty-four hours later, rats were submitted to the test swim, in which the rats were again placed in the cylinder for 5 minutes. Test swims were videotaped and subsequently assessed for latency to immobility, which is the point at which no movements were observed and the rat was in the "deadman" position *(i.e.,* head up, tail down, front legs slightly hunched, and rear legs pointed downwards). Rats were orally administered their respective doses of either 250 mg/kg body weight or 500 mg/kg body weight of HMB four hours and one hours prior to the swim test.

HMB significantly increased the latency to immobility in the forced swim test in rats, indicating antidepressant activity, as shown in Figure 1. The data was obtained from two independent experiments. The effect of HMB was consistent between the two experiments.

The complete disclosure of all patents, patent applications, and publications, and electronically available material cited herein are incorporated by reference. The foregoing detailed description and examples have been given for clarity of understanding only. No unnecessary limitations are to be understood therefrom. The invention is not limited to the exact details shown and described, for variations obvious to one skilled in the art will be included within the invention defined by the claims.

## Claims

1. A method of treating or preventing depression, comprising administering an effective amount of β**-**hydroxy-β-methylbutyrate (HMB) or a pharmaceutically acceptable salt thereof to a subject in need thereof.

2. The method of claim 1, wherein the HMB is administered orally.

3. The method of claim 1, wherein the HMB is administered as part of a nutritional composition.

4. The method of claim 3, wherein the nutritional composition contains 150-500 calories per serving and is in the form of a powder suitable for reconstitution to a liquid, a liquid or a bar.

5. The method of any one of claims 3 or 4, wherein the nutritional composition further comprises at least one source of carbohydrate and at least one source of protein.

6. The method of any one of claims 3 or 4, wherein the nutritional composition further comprises at least one source of fat, at least one source of carbohydrate, and at least one source of protein.

7. The method of claim 6, wherein the carbohydrate comprises sucrose, and the protein comprises casein.

8. The method of any one of claims 1-3, wherein the HMB comprises a calcium salt of HMB.

9. The method of any one of claims 1-3, wherein the effective amount ranges from about 0.1 g/day to about 10 g/day of HMB.

10. The method of any one of claims 1-3, wherein the effective amount ranges from 1 g/day to 10 g/day of HMB.

11. The method of any one of claims 1-3, wherein the effective amount ranges from 5 g/day to 10 g/day of HMB.

12. The method of any one of claims 1-3, wherein an effective amount of HMB is administered to the subject every day for at least two weeks.

13. The method of any one of claims 1-3, wherein the subject is a human.

14. The method of claim 13, wherein the subject is elderly.

15. A method of treating or preventing depression, comprising the daily oral administration of one or more servings of a nutritional composition, wherein the one or more servings provide 1 to 10 grams of β-hydroxy-β-methylbutyrate (HMB) or a pharmaceutically acceptable salt thereof, 150-500 calories per serving, at least one source of carbohydrate and at least one source of protein, to a human subject in need thereof for at least one week.

16. The method of claim 15, wherein the nutritional composition is in the form of a powder suitable for reconstitution to a liquid, a liquid or a bar.

17. The method of any one of claims 15 or 16, wherein 3 to 5 grams of HMB are administered.

18. The method of any one of claims 15 or 16, wherein the daily amount of HMB administered is 5 to 10 grams.

19. The method of any one of claims 15 or 16, wherein the method further comprises administering an additional antidepressant compound.

20. A method of treating a subject suffering from a condition which can be improved or prevented by the activation of mTOR signaling, comprising administering an effective amount of β-hydroxy-β-methylbutyrate (HMB) or a pharmaceutically acceptable salt thereof to a subject in need thereof.

21. The method of claim 20, wherein the effective amount ranges from about 0.1 g/day to about 10 g/day of HMB.

22. The method of claim 20, wherein the effective amount of HMB or a pharmaceutically acceptable salt thereof is administered via daily oral administration of one or more servings of a nutritional composition, wherein the one or more servings provide 1 to 10 grams of β-hydroxy-β-methylbutyrate (HMB) or a pharmaceutically acceptable salt thereof, 150-500 calories per serving, at least one source of carbohydrate and at least one source of protein, to a human subject in need thereof for at least one week.

23. The method of any one of claims 20-22, wherein the condition is selected from the group consisting of bipolar disorder, acute and chronic stress-related disorders, age-associated mood disturbances and disorders, menopause-associated mood disturbances and disorders, chronic pain syndrome, and combinations thereof.

24. The method according to claim 23, wherein the acute and chronic stress-related disorders are selected from the group consisting of generalized anxiety, post-traumatic stress disorder, phobias, panic disorder, burnout syndrome, social anxiety, jet-lag syndrome, and combinations thereof.

25. Use of β-hydroxy-β-methylbutyrate (HMB) to treat a subject suffering from a condition which can be improved or prevented by the activation of mTOR signaling.

26. The use according to claim 25, wherein the activation of mTOR signaling occurs in neuronal cells.

27. The use according to claim 25 or 26, where the condition is depression.

28. The use according to claim 25 or 26, wherein the condition is selected from the group consisting of bipolar disorder, acute and chronic stress-related disorders, age-associated mood disturbances and disorders, menopause-associated mood disturbances and disorders, chronic pain syndrome, and combinations thereof.

29. The use according to claim 28, wherein the acute and chronic stress-related disorders are selected from the group consisting of generalized anxiety, post-traumatic stress disorder, phobias, panic disorder, burnout syndrome, jet-lag syndrome, social anxiety, and combinations thereof.
